# EUROPEAN PATENT SPECIFICATION

(11) **EP 4 440 543 B1**
(45) Date of publication and mention of the grant of the patent: **16.09.2026**
(21) Application number: 22822430.9
(22) Date of filing: 25.11.2022
(51) Int. Cl.: A61K 8/99, A61Q 19/08

(54) **LACTOBACILLI FOR SKIN AGING**
LAKTOBAZILLEN FÜR DIE HAUTALTERUNG
LACTOBACILLI POUR LE VIEILLISSEMENT DE LA PEAU

(30) Priority: 30.11.2021 EP 21211430
(43) Date of publication of application: 09.10.2024
(73) Proprietor: International N&H Denmark ApS, 2800 Kgs. Lyngby (DK)
(72) Inventor: ANGLENIUS, Heli, 48210 Kotka (FI); HUUSKONEN, Laura Tiina Maria, 48210 Kotka (FI); TIIHONEN, Kirsti, 02460 Kantvik (FI)
(74) Representative: International N&H EMEA
(86) International application number: PCT/EP2022/083302
(87) International publication number: WO 2023/099351

(56) References cited:
- WO-A1-2020/001747
- WO-A1-2020/245797
- CN-A- 113 604 395
- KR-A- 20170 001 025
- KR-A- 20170 143 478
- KR-B1- 102 235 470

## Description

### FIELD OF THE INVENTION

This invention relates to new uses of a bacterium selected from strain Ligilactobacillus salivarius Ls-33 (Ls-33), strain Lacticaseibacillus paracasei Lpc-37 (Lpc-37), strain Lacticaseibacillus rhamnosus HN001 (HN001), strain Lactiplanti- bacillus plantarum Lp-12407 (Lp-12407) and strain Lactiplantibacillus plantarum Lp- 12418 (Lp-12418) for skin aging. This invention also relates to compositions, skin care compositions, food products, dietary supplements, nutritional supplements, or pharmaceutical acceptable compositions comprising said bacterium.

### BACKGROUND OF THE INVENTION

The human skin is the biggest organ of the human body and it is the barrier that separates the body from the outer environment. One of its most important roles is protecting the body from inside-out water loss and outside-in microorganism infection. The human skin consists of two main layers of cells, epidermis and dermis. Epidermis is the outermost layer of the skin and is mainly formed of terminally differentiated keratinocytes and lipids, living dividing keratinocytes located beneath the terminally differentiated ones. The main function of epidermis is to form permeability barrier against environmental challenges, such as UV radiation, heat, chemicals, pollution, and pathogens, such as bacteria, fungi, parasites, and viruses. It also protects the body from uncontrolled water evaporation from inside out, maintaining the hydration balance and skin metabolism. Epidermal tight junctions play an important role in skin barrier function being flexible and interacting with the other components in skin.

Although aging of the skin begins from the time one is born, it shows obvious and visible signs of aging when one becomes older. Skin aging can be caused by intrinsic aging or extrinsic aging. The term skin exposome describes these external and internal factors, and their interactions, that contribute to the aging of the skin. External factors, such as sun radiation (ultraviolet radiation, visible light and infrared radiation), heat, air pollution, tobacco smoke, alcohol consumption, nutrition, and a number of less well-defined miscellaneous factors, such as stress, sleep deprivation, temperature, and utilization of some cosmetic products, can contribute to the skin aging. These factors do not only affect the epidermis of the skin, but also the dermis, contributing to the aging of skin at multiple levels. Also, different disease conditions can contribute to aging. For example, the functions of tight junctions are known to be disturbed in skin diseases such as psoriasis and atopic dermatitis, as well as during aging. Skin aging is therefore a complex biological process influenced by a combination of endogenous or intrinsic and exogenous or extrinsic factors.

In the dermis, the most abundant cell type, dermal fibroblasts, are responsible of generating the connective tissue by producing extracellular matrix (ECM). This extracellular matrix (ECM) is composed of two main classes of macromolecules: proteoglycans (PGs) and fibrous proteins; the most abundant fibrous proteins are type I collagen fibrils, elastins, laminins and fibron-ectins. During aging the collagen fibrils become fragmented, fibroblasts produce less ECM proteins and more ECM degrading matrix metalloproteinases (MMPs), that leads to imbalance in the ECM.

In addition to changes in the collagen and MMPs, the following alterations can be induced in aging: nuclear and mitochondrial DNA mutations, single-stranded breaks, and oxidized pyrimidine bases are induced; membrane protein carbonylation and lipid peroxidation increase and apoptosis of epidermal keratinocytes increases, among others. Many cytokines *in vivo* peak in youth and decline thereafter. The cytokines expressed in the cells forming the different layers of the skin affect the expression of cells in neighboring tissue sections. In the question of skin, cytokines produced and secreted by the keratinocytes in the epidermis affect the cells in the dermis, or fibroblasts and their activity and extracellular matrix production, and likewise, the fibroblast-derived cytokines stimulate keratinocyte proliferation resulting in amplification of the initial signaling loop.

In addition to these changes, skin damage induced by tobacco smoking and air pollutants activate the aryl hydrocarbon receptor (AhR) pathway, a ligand-dependent transcription factor that mediates toxicity induced by several environmental contaminants, which may play a role in already known premature skin-aging effects.

Furthermore, skin barrier function is important for the protection of the skin from the external contaminants, and thus, proper function of the skin barrier is important also in preventing aging. Skin barrier is multi-cellular, with a highly organized, layered structure that provides a critical defense mechanism. In addition to lipids in the epidermis, tight junctions, hyaluronic acid, natural moisturizing factors and osmolytes in the epidermis have been shown to contribute to the overall skin barrier function. In aged human skin, there is a decrease in the expression of the tight junction components claudin-1 and occludin, and aberrant increase in claudin-6, and this reduces the tight junction barrier function. Thus, epidermal tight junction barrier of the skin is disrupted during aging, and with following insults there is decreased recovery of function, due to decrease in the homotypic cell-cell adhesion molecules or alterations in the cytokine production. Especially IL-1α production is stimulated upon injury and knocking out the receptor for this cytokine exacerbates barrier dysfunction and decreases recovery. It has been also shown that proper skin barrier function in aged mice protects from age-associated systemic inflammation, and cytokines can also affect the barrier. Depending on the cytokine, it can be proinflammatory in nature, such as IL-1, IL-6, IL-8, TNF-α, or anti-inflammatory, such as IL-10 or IL-4, and these can be secreted by various cell types, including cells in the skin. Transepidermal electrical resistance (TEER) can be used to measure epidermal tight junction integrity and skin barrier strength.

Because skin health and beauty are considered one of the principal factors representing overall "well-being" and the perception of "health" in humans, several anti-aging strategies have been developed during the last years. There is, however, a continuous need for products and methods capable of preventing, reducing or treating skin aging, not only for therapeutic reasons, but also for non-therapeutic uses, for example for cosmetic or skin care See e.g. WO 2020/245797 A1, CN 113 604 395 A, WO 2020/001747 A1, KR 2017 0143478 A, KR 2017 0001025 A, KR 102 235 470 B1.

### OBJECT OF THE INVENTION

It is an object of the present invention to provide means for preventing, reducing or treating skin aging in a subject. In particular, it is an object of the present invention to provide means to maintain metabolic activity/cell viability in the skin, to increase the metabolic activity/cell viability in the skin or slow down the reduction of metabolic activity and/or cell viability in the skin. Furthermore, it is the object of the present invention to provide means for skin rejuvenation and anti-aging purposes to maintain and stimulate epidermal skin natural and intrinsic homeostatic cytokine production of keratinocytes that decline during aging. It is yet another object of this invention to provide means to maintain and stimulate epidermal skin natural and intrinsic homeostatic cytokine production of keratinocytes to maintain metabolic activity/cell viability in the skin, to increase the metabolic activity/cell viability in the skin or slow down the reduction of metabolic activity and/or cell viability in the skin. It is yet another object of the present invention to provide means to increase and improve the structural integrity and the strength of the skin barrier by promoting the production of homeostatic cytokines of keratinocytes. It is yet another object of the present invention to provide means to maintain and stimulate epidermal skin natural and intrinsic homeostatic cytokine production of keratinocytes to affect the cells in the dermis, or fibroblasts, and their anti-aging activities and extracellular matrix production for anti-aging purposes.

### SUMMARY OF THE INVENTION

In one aspect, the invention concerns a non-therapeutic use as defined in the respective appended claims relating to a non therapeutic use.

In another aspect, the invention concerns a bacterium as defined in the appended claims for use in preventing, reducing or treating skin aging in a subject.

### BRIEF DESCRIPTION OF THE FIGURES

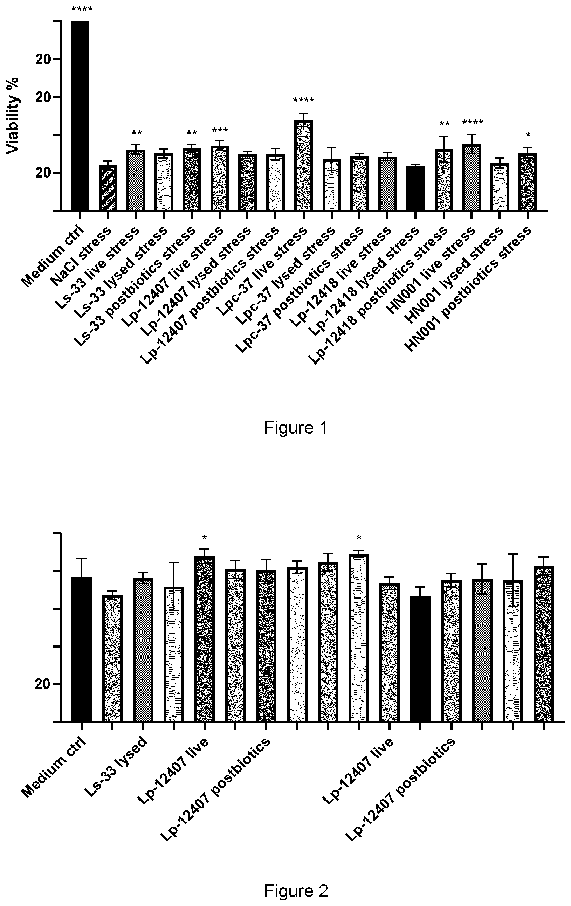

FIGURE 1 is a graph showing the effects of strains Ls-33, Lp-12407, Lpc-37, Lp-12418 and HN001 on Normal Human Epidermal Keratinocyte (NHEK) cells metabolic activity (viability) under hyper-osmotic stress (induced by NaCl), compared to NaCl stress control, when medium control values are normalized to 100 percent metabolic activity (viability). Statistical significances shown as following: ****p<0.0001, ***p<0.001, **p<0.01, *p<0.05. Ordinary one-way ANOVA, Dunnett's multiple comparisons test.
FIGURE 2 is a graph showing the effects of strains Ls-33, Lp-12407, Lpc-37, Lp-12418 and HN001 on Normal Human Epidermal Keratinocyte (NHEK) cells confluency as percent compared to medium control. Statistical significances shown as following: *p<0.05. Ordinary one-way ANOVA, Dunnett's multiple comparisons test.

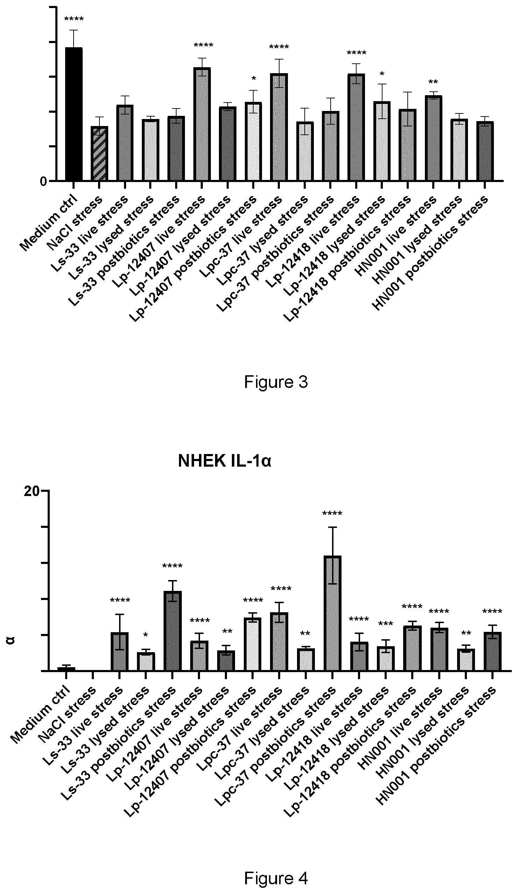

FIGURE 3 is a graph showing the effects of strains Ls-33, Lp-12407, Lpc-37, Lp-12418 and HN001 on Normal Human Epidermal Keratinocyte (NHEK) cells confluency under hyper-osmotic stress (induced by NaCl) as percent compared to NaCl stress control. Statistical significances shown as following: ****p<0.0001, **p<0.01, *p<0.05. Ordinary one-way ANOVA, Dunnett's multiple comparisons test.
FIGURE 4 is a graph showing the synthesis of IL-1α in pg/ml of strains Ls-33, Lp-12407, Lpc-37, Lp-12418 and HN001 incubated with Normal Human Epidermal Keratinocytes (NHEK) cells under hyper-osmotic stress (induced by NaCl), compared to NaCl stress control. Statistical significances shown as following: ****p<0.0001, ***p<0.001, **p<0.01, *p<0.05. Ordinary one-way ANOVA, Dunnett's multiple comparisons test.

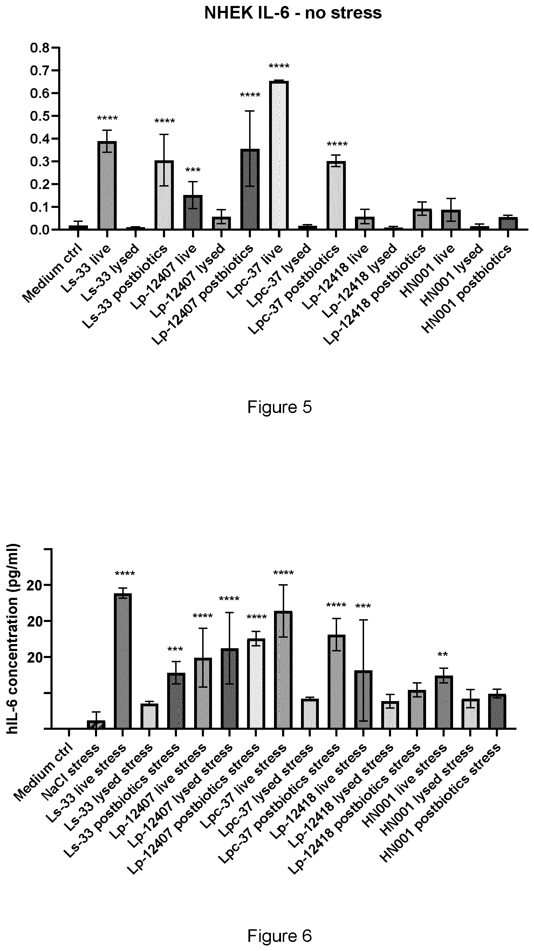

FIGURE 5 is a graph showing the synthesis of IL-6 in pg/ml of strains Ls-33, Lp-12407, Lpc-37, Lp-12418 and HN001 incubated with Normal Human Epidermal Keratinocytes (NHEK) cells compared to cell culture medium control. Statistical significances shown as following: ****p<0.0001, ***p<0.001. Ordinary one-way ANOVA, Dunnett's multiple comparisons test.
FIGURE 6 is a graph showing the synthesis of IL-6 in pg/ml of strains Ls-33, Lp-12407, Lpc-37, Lp-12418 and HN001 incubated with Normal Human Epidermal Keratinocytes (NHEK) cells under hyper-osmotic stress (induced by NaCl), compared to NaCl stress control. Statistical significances shown as following: ****p<0.0001, ***p<0.001, **p<0.01. Ordinary one-way ANOVA, Dunnett's multiple comparisons test.

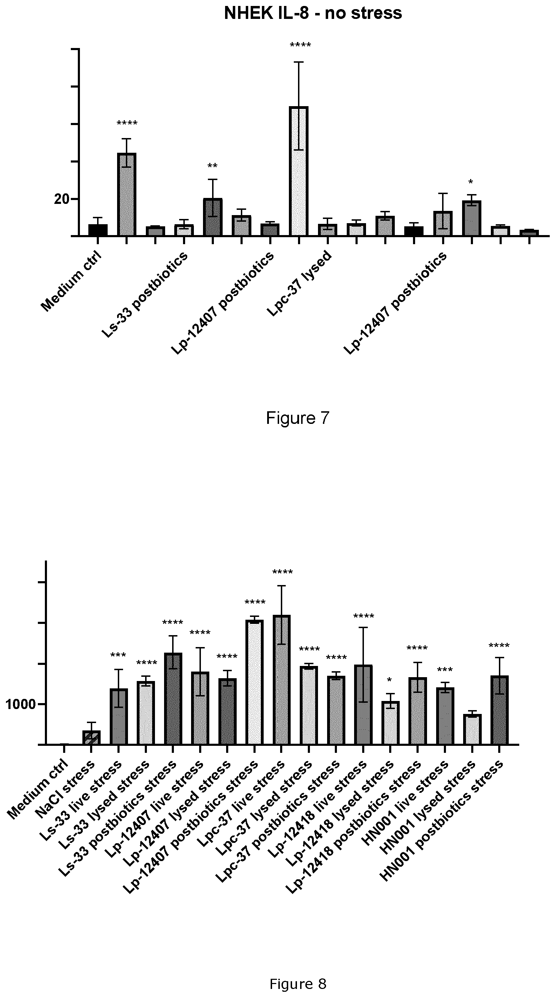

FIGURE 7 is a graph showing the synthesis of IL-8 in pg/ml of strains Ls-33, Lp-12407, Lpc-37, Lp-12418 and HN001 incubated with Normal Human Epidermal Keratinocytes (NHEK) cells compared to cell culture medium control. Statistical significances shown as following: ****p<0.0001, **p<0.01, *p<0.05. Ordinary one-way ANOVA, Dunnett's multiple comparisons test.
FIGURE 8 is a graph showing the synthesis of IL-8 in pg/ml of strains Ls-33, Lp-12407, Lpc-37, Lp-12418 and HN001 incubated with Normal Human Epidermal Keratinocytes (NHEK) cells under hyper-osmotic stress (induced by NaCl), compared to NaCl stress control. Statistical significances shown as following: ****p<0.0001, ***p<0.001, *p<0.05. Ordinary one-way ANOVA, Dunnett's multiple comparisons test.

### ADVANTAGES

In the following citation of any of a "bacterium of the species Ligilactobacillus salivarius, Lacticaseibacillus paracasei, Lac- ticaseibacillus rhamnosus, Lactiplantibacillus plantarum or a mixture thereof" is to be interpreted as being strictly limited to a "bacterium selected from strain Ligilactobacillussalivarius Ls-33 (Ls-33), strain Lacticaseibacillus paracasei Lpc-37 (Lpc-37), strain Lacticaseibacillus rhamnosus HN001 (HN001), strain Lactiplantibacillus plantarum Lp-12407 (Lp-12407) and strain Lactiplantibacillus plantarum Lp-12418 (Lp-12418), or a mixture thereof".

It has surprisingly been found by the present inventors that the use of bacterium of the species *Ligilactobacillus salivarius, Lacticaseibacillus paracasei, Lacticaseibacillus rhamnosus,* or *Lactiplantibacillus plantarum* or a mixture thereof can maintain metabolic activity/cell viability in the skin, increase the metabolic activity/cell viability in the skin, or slow down reduction of metabolic activity and/or cell viability in the skin.

It was further found out by the present inventors that bacterium of the species *Ligilactobacillus salivarius, Lacticaseibacillus paracasei, Lacticaseibacillus rhamnosus,* or *Lactiplantibacillus plantarum* or a mixture thereof can also provide means for skin rejuvenation and anti-aging purposes to maintain and stimulate epidermal skin natural and intrinsic homeostatic cytokine production of keratinocytes that decline during aging. It was discovered that bacterium of the species *Ligilactobacillus salivarius, Lacticaseibacillus paracasei, Lacticaseibacillus rhamnosus,* or *Lactiplantibacillus plantarum* or a mixture thereof can maintain and stimulate epidermal skin natural and intrinsic homeostatic cytokine production of keratinocytes to maintain metabolic activity/cell viability in the skin, to increase the metabolic activity/cell viability in the skin or slow down the reduction of metabolic activity and/or cell viability in the skin.

It was further found out that bacterium of the species *Ligilactobacillus salivarius, Lacticaseibacillus paracasei, Lacticaseibacillus rhamnosus,* or *Lactiplantibacillus plantarum* or a mixture thereof can increase and improve the structural integrity and the strength of the skin barrier by promoting the production of homeostatic cytokines of keratinocytes.

It was further discovered that bacterium of the species *Ligilactobacillus salivarius, Lacticaseibacillus paracasei, Lacticaseibacillus rhamnosus,* or *Lactiplantibacillus plantarum* or a mixture thereof can maintain and stimulate natural and intrinsic homeostatic cytokine production of keratinocytes to affect the cells in the dermis, or fibroblasts and their anti-aging activities and extracellular matrix production for anti-aging purposes. This shows the ability for bacterium of the species *Ligilactobacillus salivarius, Lacticaseibacillus paracasei, Lacticaseibacillus rhamnosus,* or *Lactiplantibacillus plantarum* or a mixture thereof to prevent, reduce or treat skin aging in a subject.

### DETAILED DISCLOSURE OF THE INVENTION

### Bacteria

The bacteria used in the present invention are selected from bacteria of the genus *Lactobacillus.*

Preferably the *Lactobacillus* to be used in the present invention is a *Lactobacillus* which is generally recognised as safe and, which is preferably GRAS approved. Generally recognized as safe (GRAS) is an American Food and Drug Administration (FDA) designation that a chemical or substance added to food is considered safe by experts, and so is exempted from the usual Federal Food, Drug, and Cosmetic Act (FFDCA) food additive tolerance requirements.

Preferably, the bacterial strains of the present invention are selected from bacterial strains of the species *Ligilactobacillus salivarius, Lacticaseibacillus paracasei, Lacticaseibacillus rhamnosus,* or *Lactiplantibacillus plantarum* or a mixture thereof.

In one embodiment, the present invention relates to a non-therapeutic use of a bacteria(um) of the species *Ligilactobacillus salivarius, Lacticaseibacillus paracasei, Lacticaseibacillus rhamnosus,* or *Lactiplantibacillus plantarum* or a mixture thereof for preventing, reducing or treating skin aging in a subject.

The term "subject", as used herein, means an animal having a skin that separates and defines the animal inside from the outside. Preferably, the subject is a mammal.

In a particular aspect of the present invention, the subject may suitably be a human.

In one embodiment the subject may be female.

In one embodiment the subject may be male.

In one embodiment the subject may be with a non-binary gender.

In one embodiment the subject is not a child. The term "child" as used herein means a human 7 years of age or younger.

In one embodiment the subject is a human that is 8 years of age or older.

In one embodiment the subject is a human that is 16 years of age or older.

In one embodiment the subject is a human that is 18 years of age or older.

In one embodiment the subject is a healthy subject.

In one embodiment, the subject suffers from cancer, chronic inflammation, skin infection or any other transient or chronic medical condition responsible for skin aging.

A non-therapeutic use includes, but it is not limited to, cosmetic or skin care uses or purposes. Therefore, the invention described herein also relates to a cosmetic or skin care use of a bacterium of the species *Ligilactobacillus salivarius, Lacticaseibacillus paracasei, Lacticaseibacillus rhamnosus,* or *Lactiplantibacillus plantarum* or a mixture thereof for preventing, reducing or treating skin aging in a subject.

In an embodiment, the bacteria(um) of the species *Ligilactobacillus salivarius, Lacticaseibacillus paracasei, Lacticaseibacillus rhamnosus,* or *Lactiplantibacillus plantarum* or the mixture thereof maintains metabolic activity/cell viability in the skin, increases the metabolic activity/cell viability in the skin or slows down the reduction of metabolic activity and/or cell viability in the skin.

Keratinocytes are able to detect broad range of small molecular motifs present on bacteria and other microorganisms, which are referred as pathogen-associated molecular patterns (PAMPs), and these are present in both gram-positive and gram-negative bacteria. The recognition of the PAMPs occurs through pattern recognition receptors, such as toll-like receptors (TLRs). Thus, keratinocytes are part of first line of defense mechanisms or innate immunity and produce factors crucial in homeostasis and in tissue repair, and elicit immune responses depending on the activating PAMP and activating receptor. By secreting cytokines and chemokines, keratinocytes can recruit, activate, and regulate immune cells. Not all the signals are causing inflammation but acting stimulatory and preparing and educating the immune system for possible encounter of pathogens, limiting tissue inflammation and microbial translocation. This can be achieved by integrating the signals from microbe-derived products to epithelial cells that integrate these multiple signals in order to ensure barrier integrity and tissue homeostasis. Inflammation is important for skin repair processes as well, such as during wound healing, and therefore, targeting inflammatory responses to improve healing outcomes and skin regeneration are desired.

The function and expression of the structural proteins involved in the skin barrier formation are highly regulated in a differentiation associated manner by cytokines and other signaling molecules. Defects in the skin barrier and desquamation of corneocytes can result in disturbed epidermal barrier, leading to entry of environmental harmful contaminants, allergens, and pathogens to the skin. Also, keratinocytes express various immune markers, cytokines and effector molecules when activated by an injury, such as during wound healing, or together with external pathogens. While the hyperosmotic stress encountered by the keratinocytes is part of the normal differentiation process, it is also experienced under pathological conditions. It may contribute to the formation of irritant contact dermatitis which is frequent occupational disorder. Also, UV radiation causes oxidative stress which disturb the ionic regulation in the keratinocytes and causes cell shrinkage and hyperosmotic stress. Hyperosmotic stress has been shown to induce multiple changes in the cells, and it can prime the cells for apoptosis, and inhibits the cell proliferation. When induced with 0.5 M NaCl in NHEKs, it has been shown to be proinflammatory and increase expression of cytokines such as IL-1a, IL-1β, IL-6, IL-8 but not IL-10. In the invention, the stress was induced with 0.15 M (150 mM) NaCl to provide a milder stimulus not inducing cell death.

Skin aging, like aging in other organs, is characterized by progressive loss of skin cell functionality and regenerative potential. There are similarities between effects of intrinsic and extrinsic aging. Most obvious change during aging of the skin is the thinning of skin and impairment of skin recovery and loss of turnover or senescence and structural changes that occur over time. By promoting the proliferation, the production of cytokines stimulated by the lactobacilli participate in the skin homeostasis by increasing the viability and proliferation, and through this enhance the skin's natural renewal process capacity that declines during aging skin. By controlling homeostatic immunity, these lactobacilli strains enhance the natural defense mechanisms towards pathogenesis and various environmental factors that enhance the skin aging.

Cytokines are added in several cosmetic products as anti-ageing components, and in our invention, the probiotics regulate the natural and intrinsic cytokine production of keratinocytes for skin rejuvenation and anti-aging purpose. Aging is, in many respects, related to wound healing in a manner that it overcomes the skin's inherited repair mechanisms that decline during aging, and relevant cytokines in wound healing have been shown to be important also in skin aging. By administering topical or by ingesting lactobacilli strains, the skin's own production of cytokines is stimulated to slowing down or even reversing the manifestation of the skin aging. Thus, in skin keratinocytes the lactobacilli strains described can improve the structural integrity and the strength of the skin barrier by promoting the production of homeostatic cytokines.

In another embodiment of the present invention, the bacteria(um) of the species *Ligilactobacillus salivarius, Lacticaseibacillus paracasei, Lacticaseibacillus rhamnosus,* or *Lactiplantibacillus plantarum* or the mixture thereof maintain(s) and stimulate(s) epidermal skin natural and intrinsic homeostatic cytokine production of keratinocytes.

Aging is, in many respects, related to wound healing in a manner that it overcomes the skin's inherited repair mechanisms that decline during aging, and relevant cytokines in wound healing have been shown to be important also in skin aging. Therefore, the bacterium of the species *Ligilactobacillus salivarius, Lacticaseibacillus paracasei, Lacticaseibacillus rhamnosus,* or *Lactiplantibacillus plantarum* or the mixture thereof provide means for skin rejuvenation and anti-aging purposes to maintain and stimulate epidermal skin natural and intrinsic homeostatic cytokine production of keratinocytes that decline during aging.

The maintenance and stimulation of the natural and intrinsic homeostatic cytokine production of keratinocytes affects the cells in the dermis, or fibroblasts and their anti-aging activities and extracellular matrix production.

According to the present invention, the maintenance and stimulation of the epidermal skin natural and intrinsic homeostatic cytokine production of keratinocytes maintains a metabolic activity/cell viability in the skin, increases the metabolic activity/cell viability in the skin or slow down the reduction of metabolic activity and/or cell viability in the skin.

In a further embodiment of the present invention, the bacteria(um) of the species *Ligilactobacillus salivarius, Lacticaseibacillus paracasei, Lacticaseibacillus rhamnosus,* or *Lactiplantibacillus plantarum* or the mixture thereof increases and improves the structural integrity and the strength of the skin barrier. The increase and improvement of the structural integrity and the strength of the skin barrier is achieved by the production of homeostatic cytokines of keratinocytes.

In particular, the bacteria(um) of the present invention are chosen from the strains Ls-33, Lpc-37, HN001, Lp-12407 and Lp12418.

The strains can be used individually or in combination, for example, in skin care compositions, dietary supplements, nutritional supplements, food products or the pharmaceutical acceptable compositions.

The bacterial strains of the present invention are all commercially available from DuPont Nutrition Biosciences ApS.

The bacterial strains were also deposited by DuPont Nutrition Biosciences ApS, of Langebrogade 1, DK-1411 Copenhagen K, Denmark, in accordance with the Budapest Treaty at the Leibniz-Institut Deutsche Sammlung von Mikroorganismen und Zellkulturen GmbH (DSMZ), Inhoffenstrasse 7B, 38124 Braunschweig, Germany, where they are recorded under the following registration numbers:
1. Strain Ls-33 (DGCC9868); deposited on 23 February 2021 under registration number DSM33831.
2. Strain Lpc-37 (DGCC4981); deposited on 5 October 2017 under registration number DSM32661.
3. Strain HN001 (DGCC1460); deposited on 7 May 2021 under registration number DSM 22876.
4. Strain Lp12418 (DGCC12418); deposited on 27 September 2017 under registration number DSM32655.
5. Strain Lp-12407 (DGCC12407); deposited on 27 September 2017 under registration number DSM32654.

In a particular embodiment according to the present invention, the bacterium of the species *Ligilactobacillus salivarius* is strain *Ligilactobacillus salivarius* Ls-33 (Ls-33).

In another embodiment, the bacterium of the species *Lacticaseibacillus paracasei* is strain *Lacticaseibacillus paracasei* Lpc-37 (Lpc-37).

In a further embodiment, the bacterium of the species *Lacticaseibacillus rhamnosus* is strain *Lacticaseibacillus rhamnosus* HN001 (HN001).

In another embodiment, the bacterium of the species *Lactiplantibacillus plantarum* is strain *Lactiplantibacillus plantarum* Lp-12407 (Lp-12407) and or strain *Lactiplantibacillus plantarum* Lp-12418 (Lp-12418).

In the dermis, the most abundant cell type, dermal fibroblasts, are responsible of generating the connective tissue by producing extracellular matrix (ECM). During aging the collagen fibrils become fragmented, fibroblasts produce less ECM proteins and more ECM degrading matrix metalloproteinases (MMPs), that leads to imbalance in the ECM. In a further embodiment, the prevention, reduction or treatment of the skin aging in a subject is attained by maintaining the metabolic activity/cell viability in the skin, increasing it or slowing down the reduction of metabolic activity and/or cell viability in the skin and/or by increasing or maintaining natural and intrinsic homeostatic cytokine production of keratinocytes to affect the cells in the dermis, or fibroblasts, and their anti-aging activities and extracellular matrix production for anti-aging purposes.

In a further embodiment, the present invention relates to skin aging caused by intrinsic and extrinsic factors, such as, but not limited to, oxidative damage, DNA damage, impaired DNA repair, impaired cell division, excessive inflammation, inflammaging, immune diseases, excessive cell death, sun, sunburn, collagen damage, elastin damage, senescence, telomere shortening, impaired expression of antioxidant enzymes, impaired activity of antioxidant enzymes, infrared or UV radiation, heat, hormonal reasons, poor nutrition, dehydration, temperature, tobacco smoking, stress, sleep deprivation, pollution, or alcohol consumption.

In one embodiment, the bacterium used in the present invention is a probiotic bacterium. In this specification the term "probiotic bacterium" is defined as covering any non-pathogenic bacterium which, when administered live, for example orally or topically, in adequate amounts, confer a health benefit on the host. Also, probiotic strains generally have an ability to survive the passage through the upper part of the digestive tract when intended for oral use. They are non-pathogenic, non-toxic and exercise their beneficial effect on health on the one hand via ecological interactions with the resident flora, and on the other hand via their ability to influence the immune system in a positive manner via the "MALT" (mucosa-associated lymphoid tissue).

Probiotics can form part of the resident flora during the administration period. This colonization (or transient colonization) allows the probiotic bacteria to exercise a beneficial effect, such as normalization of perturbed microbiota by for instance through repression of potentially pathogenic bacteria, resisting their colonization, or competitive exclusion, and interactions with the immune system of the intestine or modulation of the fermentation of the gut microbiota. The probiotics mediate the health benefit also through production of enzymes or vitamins or other bioactives, through modulation of bile acid metabolism, and neutralization of harmful metabolites produced by other microbes.

In preferred embodiments, the bacterium used in the present invention is a probiotic *Lactobacillus.*

The bacteria may be used in any form capable of exerting the effects described herein. For example, the bacteria may be viable, dormant, inactivated, lysed or dead bacteria. Preferably, the bacteria are in live form.

In a preferred embodiment, the bacteria are in a lysed form.

The bacteria may comprise whole bacteria or may comprise bacterial components. Examples of such components include, but not limited to, bacterial cell wall components such as peptidoglycan, lipoteichoic acid, bacterial nucleic acids such as DNA and RNA, bacterial membrane components, and bacterial structural components such as proteins, carbohydrates, lipids and combinations of these such as lipoproteins, glycolipids and glycoproteins.

The bacteria may also or alternatively comprise bacterial metabolites. In the present specification the term "bacterial metabolites" includes all molecules produced or modified by the bacteria as a result of bacterial metabolism during growth, survival, persistence, transit or existence of bacteria during the manufacture of the probiotic product and storage and during gastrointestinal transit in a mammal. Examples include, but not limited to, all organic acids, inorganic acids, bases, proteins and peptides, enzymes and co-enzymes, amino acids and nucleic acids, carbohydrates, lipids, glycoproteins, lipoproteins, glycolipids, vitamins, all bioactive compounds, metabolites containing an inorganic component, and all small molecules, for example nitrous molecules or molecules containing a sulphurous acid.

In another preferred embodiment, the bacteria are in a postbiotic form.

Postbiotics refers to products or metabolic byproducts, or metabolites, secreted by live bacteria generated through fermentation to the matrix, or released after bacterial lysis, such as, but not limited to, microbial cell fractions, proteins, enzymes, peptides, teichoic acids, peptidoglycan-derived muropeptides, polysaccharides, extracellular polysaccharides, cell surface proteins, pili-like structures, lipids and organic acids. These postbiotics because of their clear chemical structure, safety dose parameters, long shelf life and the content of various signaling molecules present anti-inflammatory, immunomodulatory, anti-obesogenic, antihypertensive, hypocholesterolemic, anti-proliferative, and/or antioxidant properties.

The invention further provides a mutant, a variant and/or a progeny of the bacterial strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418).

As used herein, the term "mutant" refers to any microorganism resulting from modification of the strains *Ligilactobacillus salivarius* Ls-33 (Ls-33), *Lacticaseibacillus paracasei* Lpc-37 (Lpc-*37), Lacticaseibacillus rhamnosus* HN001 (HN001), *Lactiplantibacillus plantarum* Lp-12407 (Lp-12407) and *Lactiplantibacillus plantarum* Lp-12418 (Lp-12418). For example, a mutant may be a microorganism resulting from genetically modifying these strains.

As used herein, the term "variant" refers to a naturally occurring microorganism which is derived from the strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418). For example, a variant may be a microorganism resulting from adaption to a particular environment or cell culture conditions.

As used herein, the term "progeny" means any microorganism resulting from the reproduction or multiplication of any one of the strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418). Therefore, "progeny" means any direct descendant of any one of these strains. As such, the progeny strain may itself be identified as the same strain as the parent strain (i.e. strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418)). It will be apparent to one skilled in the art that due to the process of asexual reproduction, a progeny strain will be genetically virtually identical to the parent strain. Accordingly, in one embodiment, the progeny may be genetically identical to the parent strain and may be considered to be a "clone" of the parent strain. Alternatively, the progeny may be substantially genetically identical to the parent strain.

The mutant, variant or progeny may have at least 90, 95, 98, 99, 99.5 or 99.9% sequence identity over the entire length of the bacterial genome with their parent strain. Furthermore, the mutant, variant or progeny will retain the same phenotype as the deposited parent strain, for example the mutant, variant or progeny may demonstrate the same or equivalent effect on *in vitro* cell viability, cell confluency, and maintaining or stimulating natural and homeostatic cytokine production.

For the purpose of the present invention, any mutant, variant and/or progeny of the bacterial strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) are considered to be the same as strains *Ligilactobacillus salivarius* Ls-33 (Ls-33), *Lacticaseibacillus paracasei* Lpc-37 (Lpc-*37), Lacticaseibacillus rhamnosus* HN001 (HN001), *Lactiplantibacillus plantarum* Lp-12407 (Lp-12407) and *Lactiplantibacillus plantarum* Lp-12418 (Lp-12418), respectively.

### Dosage

The *Lactobacillus,* such as species of *Ligilactobacillus salivarius, Lacticaseibacillus paracasei, Lacticaseibacillus rhamnosus,* or *Lactiplantibacillus plantarum,* for example strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) used in accordance with the present invention may be present from 10⁶ to 10¹² CFU of bacteria/g of support, and more particularly from 10⁸ to 10¹² CFU of bacteria/g of support, preferably 10⁹ to10¹² CFU/g of support.

Suitably, the genus *Lactobacillus,* such as species of *Ligilactobacillus salivarius, Lacticaseibacillus paracasei, Lacticaseibacillus rhamnosus,* or *Lactiplantibacillus plantarum,* for example strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418), may be administered at a dosage of from about 10⁶ to about 10¹² CFU of microorganism/dose,preferably about 10⁸ to about 10¹² CFU of microorganism/dose. By the term "per dose" it is meant that this amount of microorganism is provided to a subject either per day or per intake, preferably per day. For example, if the microorganism is to be administered in a food product, for example in a yoghurt, then the yoghurt will preferably contain from about 10⁸ to 10¹² CFUof the microorganism. Alternatively, however, this amount of microorganism may be split intomultiple administrations each consisting of a smaller amount of microbial loading - so long as the overall amount of microorganism received by the subject in any specific time, for instanceeach 24-hour period, is from about 10⁶ to about 10¹² CFU of microorganism, preferably 10⁸ toabout 10¹² CFU of microorganism.

In accordance with the present invention an effective amount of at least one strain of a microorganism may be at least 10⁶ CFU of microorganism/dose, preferably from about 10⁶ to about 10¹² CFU of microorganism/dose, preferably about 10⁸ to about 10¹² CFU of microorganism/dose.

In one embodiment, preferably the genus *Lactobacillus,* such as species of *Ligilactobacillus salivarius, Lacticaseibacillus paracasei, Lacticaseibacillus rhamnosus,* or *Lactiplantibacillus plantarum,* for example strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) may be administered at a dosage of from about 10⁶ to about 10¹² CFU of microorganism/day, preferably about 10⁸ to about 10¹² CFU of microorganism/day. Hence, theeffective amount in this embodiment may be from about 10⁶ to about 10¹² CFU of microorganism/day, preferably about 10⁸ to about 10¹² CFU of microorganism/day.

CFU stands for "colony-forming units". By "support" is meant a composition, a skin care composition, a dietary supplement, a nutritional supplement, a food product or a pharmaceutically acceptable composition.

In one embodiment, the present invention relates to bacteria of the genus *Lactobacillus,* such as species of *Ligilactobacillus salivarius, Lacticaseibacillus paracasei, Lacticaseibacillus rhamnosus,* or *Lactiplantibacillus plantarum,* for example strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) as at least one of the components of a composition, a skin care composition, dietary supplements, nutritional supplements, food products or a pharmaceutical acceptable composition.

### Compositions

While is it possible to administer *Lactobacilli* alone according to the present invention (i.e. without any support, diluent or excipient), the *Lactobacilli* are typically and preferably administered on or in a support as part of a product, in particular as a component or at least as one of the components of a composition, a skin care composition, a dietary supplement, a nutritional supplement, a food product or a pharmaceutical acceptable composition. These products typically contain additional components well known to those skilled in the art.

In one embodiment, the present invention relates to a composition, a skin care composition or a pharmaceutical acceptable composition for oral administration or topical administration. A composition for topical application is a composition that is applied to a particular place on or in the body. Most often topical application or administration means application to body surfaces such as the skin or mucous membranes to treat certain ailments and, for example, to prevent, reduce or treat skin aging. The topical application can be achieved using a large range of products as support, such as, but not limited to, lotions, serums, jellies, creams, gels, emulsions, masks, patches, micellar water, sticks or ointments.

In another embodiment, the present invention relates to a composition, a skin care composition, a dietary supplement, a nutritional supplement a food product or pharmaceuticalacceptable composition comprising a bacterium selected from strain Ligilactobacillussalivarius Ls-33 (Ls-33), strain Lacticaseibacillus paracasei Lpc-37 (Lpc-37), strain Lacticaseibacillus rhamnosus HN001 (HN001), strain Lactiplantibacillus plantarum Lp-12407 (Lp-12407) and strain Lactiplantibacillus plantarum Lp-12418 (Lp-12418), or a mixture thereof for oral administration.

A dietary supplement is any product intended to supplement the diet of a subject when taken orally as a pill, capsule, tablet, or liquid form.

### Skin care compositions

The present invention also relates to bacteria of the genus *Lactobacillus,* such as strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) in the form of a skin care compositions or products including, but not limited to, aqueous solutions, emulsions, serums, jellies, masks, patches, face masks, peel-off masks, lotions, topical moisturizers, creams, pastes, balms, ointments, pomades, gels, liquids, sprays, foam and kits.

A skin care product, can also be in the form of makeup, lipstick, mascara, foundation, blush, eyeliner, lip gloss, macro emulsion, sunscreen, sun block, bath gel, shower gel, body wash, face wash, skin conditioner, cold cream, moisturizer, body spray, soap and body scrub.

A skin care composition or product may include a liquid lotion (true solution) comprising water as a solvent and water-soluble additives (solutes), such as but not limiting to an active, a fragrance, a color, a preservative, a pH adjuster, a chelating agent, or any one combination thereof.

A skin care product may also include a dispersion, for example an emulsion, such as, but not limited to: liquid in liquid (water in oil W/O, O/W, W/O/W), suspension (solid/liquid or liquid/solid), aerosol (liquid/gas or solid/gas), foam/mousse (gas/liquid or gas/emulsion, or gas/solid). An example of an Oil in Water (O/W) emulsion includes but is not limited to a combination of a water phase, an emulsifier, a fatty phase and an at least one additive. The water phase can comprise water, humectants and stabilizing agents, such as, but not limiting to, synthetic polymers, carbomers, natural polymers, xanthan gum, acacia gum, carrageenan, gellan, or any one combination thereof. Emulsifiers include, but are not limited to, anionic emulsifiers, cationic emulsifiers, non-ionic emulsifiers, amphoteric emulsifiers, silicone emulsifiers, autoemulsifying agents. Fatty phases (lipophilic ingredients) include, but are not limited to, waxes, butter, fatty esters, triglycerides, vegetal oil, mineral oil (paraffinum), silicones, and thickeners/oil jellifying agents. Additives include, but are not limited to, preservative, fragrance (most often lipophilic), color, antioxidant, chelating agent, actives, pH adjuster (citric acid, lactic acid, AHA), neutralizers/strong basic agent like NaOH, Trimethylamine (for acrylic polymers to jellify) and powders.

A skin care product includes an aqueous gel comprising a water phase (including water, humectants, actives), a jellifying agent (such as but not limited to synthetic polymers, natural polymers, xanthan gum, acacia gum, carrageenan, gellan) and an additive (such as but not limited to fragrance, high HLB surfactant, color, actives, preservative system, pH adjuster, neutralizing agent, powders).

A skin care product includes a cleansing / surfactant system (such as but not limited to a shampoo, shower gel, micellar water) comprising a water phase (water, humectants), a surfactant, an additive (such as but not limited to fragrance, high HLB surfactant, color, actives,preservative system, pH adjuster, neutralizing agent, powders) and optionally a jellifying agent (such as but not limited to synthetic polymers, natural polymers, xanthan gum, acacia gum, carrageenan, gellan).

In one embodiment, a skin care product or formulation comprising an effective amount of a skin care composition, further comprises a bacterium of the species *Ligilactobacillus salivarius, Lacticaseibacillus paracasei, Lacticaseibacillus rhamnosus,* or *Lactiplantibacillus plantarum* or a mixture thereof.

In another embodiment, the skin product or formulation comprising an effective amount of a skin care composition, further comprises a bacterium of the strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) and any mixture thereof.

The skin product or formulation comprising a bacterium of the species *Ligilactobacillus salivarius, Lacticaseibacillus paracasei, Lacticaseibacillus rhamnosus,* or *Lactiplantibacillus plantarum* provides a skin care benefit by preventing, reducing or treating skin aging by maintaining the metabolic activity/cell viability in the skin, increasing it, or slowing down the reduction of metabolic activity and/or cell viability in the skin; provides means for skin rejuvenation and anti-aging purposes to maintain and stimulate epidermal skin natural and intrinsic homeostatic cytokine production of keratinocytes that decline during aging; maintains and stimulates epidermal skin natural and intrinsic homeostatic cytokine production of keratinocytes to maintain metabolic activity/cell viability in the skin, to increase the metabolic activity/cell viability in the skin or slow down the reduction of metabolic activity and/or cell viability in the skin; increases and improves the structural integrity and the strength of the skin barrier by promoting the production of homeostatic cytokines of keratinocytes; maintains and stimulates epidermal skin natural and intrinsic homeostatic cytokine production of keratinocytes to affect the cells in the dermis, or fibroblasts and their anti-aging activities and extracellular matrix production for anti-aging purposes.

The bacterium of the strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) or a mixture thereof, used in accordance with the present invention, may be present from 0.1% to 10 % on a weight basisrelative to a total weight of said skin care product. In one aspect, the preferable effective amount of the skin care composition described herein can be at least about 0.1% to 5% on a weight basis relative to a total weight of said skin care product. In one aspect, the effective amount of the skin care composition described herein can be at least about 0.1% to 10% on aweight basis relative to a total weight of said skin care product. The dermatologically acceptablecomponent can be a dermatologically acceptable carrier comprising about 10% to about 99% on a weight basis relative to a total weight of the skin care product. In one aspect at least about 0.1 %, 1%, 2%, 3%, 4%, 5%, 6%, 7%, 8%, 9% up to 10% is the live bacteria, non- viable bacteria, bacterial lysate or postbiotic described herein on a weight basis relative to a total weight of said skin care product. The effective amount of the skin care composition in saidskin care product can be at least about 0.1% to 5%, at least about 0.1% to 6%, at least about0.1% to 7%, at least about 0.1% to 8%, at least about 0.1% to 9%, at least about 0.1% to 10% on a weight basis relative to a total weight of said formulation or skin care product. In one aspect the effective amount of the skin care composition in said skin care product can be at least about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, to 10% on a weight basis relative to a total weight of said formulation or skin care product.

The skin care products described herein may further comprise one or more dermatologically orcosmetically acceptable components known or otherwise effective for use skin care, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004.

In one aspect, the dermatologically or cosmetically acceptable component is a dermatologically acceptable carrier comprising from about 10 wt.% to about 99.9 wt.%, alternatively from about 50 wt.% to about 95 wt.%, and alternatively from about 75 wt.% to about 95 wt.%, of a dermatologically acceptable carrier. Carriers suitable for use with the composition(s) may include, for example, those used in the formulation of mousses, tonics, gels, skin moisturizers and lotions. The carrier may comprise water; organic oils; silicones such as volatile silicones, amino or non-amino silicone gums or oils, and mixtures thereof; mineral oils; plant oils such as olive oil, castor oil, rapeseed oil, coconut oil, wheatgerm oil, sweet almond oil, avocado oil, macadamia oil, apricot oil, safflower oil, candlenut oil, false flax oil, tamanu oil, lemon oil and mixtures thereof; waxes; and organic compounds such as C2-C10 alkanes, acetone, methyl ethyl ketone, volatile organic C1-C12 alcohols, esters of C1-C20 acids and of C1-C8 alcohols such as methyl acetate, butyl acetate, ethyl acetate, and isopropyl myristate, dimethoxyethane, diethoxyethane, C10-C30 fatty alcohols such as lauryl alcohol, cetyl alcohol, stearyl alcohol, and behenyl alcohol; C10-C30 fatty acids such as lauric acid and stearic acid; C10-C30 fatty amides such as lauric diethanolamide; C10-C30 fatty alkyl esters such as C10-C30 fatty alkyl benzoates; hydroxypropylcellulose, and mixtures thereof. In one aspect, the carrier comprises water, fatty alcohols, volatile organic alcohols, and mixtures thereof. Other carriers can be formulated by those of ordinary skill in the art.

The skin care products described herein may further comprise from about 0.1% to about 10%, and alternatively from about 0.2% to about 5.0%, of a gelling agent to help provide the desired viscosity to the composition(s). Non-limiting examples of suitable optional gelling agents include crosslinked carboxylic acid polymers; unneutralized crosslinked carboxylic acid polymers; unneutralized modified crosslinked carboxylic acid polymers; crosslinked ethylene/maleic anhydride copolymers; unneutralized crosslinked ethylene/maleic anhydride copolymers (e.g., EMA 81 commercially available from Monsanto); unneutralized crosslinked alkyl ether/acrylate copolymers (e.g., SALCARE^{™} SC90 commercially available from Allied Colloids); unneutralized crosslinked copolymers of sodium polyacrylate, mineral oil, and PEG 1 trideceth-6 (e.g., SALCARE^{™} SC91 commercially available from Allied Colloids); unneutralized crosslinked copolymers of methyl vinyl ether and maleic anhydride (e.g., STABILEZE^{™} QM-PVM/MA copolymer commercially available from International Specialty Products); hydrophobically modified nonionic cellulose polymers; hydrophobically modified ethoxylate urethane polymers (e.g., UCARE^{™} Polyphobe Series of alkali swellable polymers commercially available from Union Carbide); and combinations thereof. In this context, the term "unneutralized" means that the optional polymer and copolymer gelling agent materials contain unneutralized acid monomers.

The cosmetically acceptable medium may contain a fatty substance in a proportion generally of from about 10 to about 90% by weight relative to the total weight of the product, where the fatty phase containing at least one liquid, solid or semi-solid fatty substance. The fatty substance includes, but is not limited to, oils, waxes, gums, and so-called pasty fatty substances. Alternatively, the products may be in the form of a stable dispersion such as a water-in-oil or oil-in-water emulsion. Additionally, the skin care products may contain one or more conventional cosmetic or dermatological additives or adjuvants, including but not limited to, antioxidants, preserving agents, fillers, surfactants, UVA and/or UVB sunscreens, fragrances, thickeners, wetting agents and anionic, nonionic or amphoteric polymers, and dyes or pigments (colorant agents).

The dermatologically acceptable carrier may be a moisturizer formulation containing at least one emulsifier, at least one surfactant, or any combination thereof.

Skin care products can further comprise skin care active ingredient materials including sunscreen agents, moisturizers, humectants, benefiting agents skin, depositing agents such as surfactants, occlusive agents, moisture barriers, lubricants, emollients, anti-aging agents, antistatic agents, abrasive, antimicrobials, conditioners, exfoliants, fragrances, viscosifying agents, salts, lipids, phospholipids, vitamins, foam stabilizers, pH modifiers, preservatives, suspending agents, silicone oils, silicone derivatives, essential oils, oils, fats, fatty acids, fatty acid esters, fatty alcohols, waxes, polyols, hydrocarbons, and mixtures thereof.

Other ingredients that may be included in a skin care product include, without limitation, at least one active ingredient for the treatment or prevention of skin ailments, providing a cosmetic effect, or for providing a moisturizing benefit to skin, such as zinc oxide, petrolatum,white petrolatum, mineral oil, cod liver oil, lanolin, dimethicone, hard fat, vitamin A, allantoin,calamine, kaolin, glycerin, or colloidal oatmeal, and combinations of these, one or more natural moisturizing factors (such as ceramides, hyaluronic acid, glycerin, squalane, amino acids, cholesterol, fatty acids, triglycerides, phospholipids, glycosphingolipids, urea, linoleic acid, gly-cosaminoglycans, mucopolysaccharide, sodium lactate, or sodium pyrrolidone carboxylate, for example), glycerides, apricot kernel oil, canola oil, squalane, squalene, coconut oil, corn oil, jojoba oil, jojoba wax, lecithin, olive oil, safflower oil, sesame oil, shea butter, soybean oil, sweet almond oil, sunflower oil, tea tree oil, shea butter, palm oil, cholesterol, cholesterolesters, wax esters, fatty acids, and orange oil.

### Food Product

In one embodiment, the *Lactobacilli* are employed according to the invention in a food product, such as a food supplement, a drink or a powder based on milk. Here, the term "food" is used in a broad sense and covers food for humans as well as food for animals (i.e. a feed). In a preferred aspect, the food is for human consumption.

The food may be in the form of a solution or as a solid, depending on the use and/or the mode of application and/or the mode of administration.

When used as, or in the preparation of, a food, such as functional food, the bacteria of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

By way of example, the bacteria of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, vegetable milk, acidified soy/juice beverage, aseptic/retorted chocolate drink, bar mixes, beverage powders, calcium fortified soy/plain and chocolate milk, calcium fortified coffee beverage. Advantageously, where the product is a food product, the *Lactobacilli* should remain effective through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.

### Medical Food

In one embodiment, the bacterium of the present invention is in the form of a medical food product.

Preferably, the strains *Ligilactobacillus salivarius* Ls-33 (Ls-33), *Lacticaseibacillus paracasei* Lpc-37 (Lpc-37), *Lacticaseibacillus rhamnosus* HN001 (HN001), *Lactiplantibacillus plantarum* Lp-12407 (Lp-12407) and *Lactiplantibacillus plantarum* Lp-12418 (Lp-12418) are in the form of a medical food product.

By "medical food" it is meant a food product which is formulated to be consumed or administered with or without the supervision of a physician and which is intended for a specificskin condition or other medical condition for which distinctive nutritional requirements, based on recognized scientific principles, are established.

### Pharmaceutical composition

The bacteria of the present invention may be used as - or in the preparation of - a pharmaceutical composition. Here, the term "pharmaceutical" is used in a broad sense - and covers pharmaceuticals for humans as well as pharmaceuticals for animals (i.e. veterinary applications).

In a preferred embodiment, the pharmaceutical acceptable composition is a medicament. The pharmaceutical composition can be for therapeutic purposes - which may be curative or palliative or preventative in nature. The pharmaceutical composition may even be for diagnostic purposes.

In a preferred embodiment of the present invention, the medicament is for topical application.

In another preferred embodiment of the present invention, the medicament is for oraladministration.

A pharmaceutically acceptable composition or support may be for example a formulation or support in the form of creams, foams, gels, lotions, and ointments of compressed tablets, tablets, capsules, ointments, suppositories or drinkable solutions. Other suitable forms are provided below.

When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.

The pharmaceutical may be in the form of a solution or as a solid - depending on the use and/or the mode of application and/or the mode of administration.

The lactobacilli of the present invention may be used as pharmaceutical ingredients. Here, the composition may be the sole active component, or it may be at least one of a number (i.e. 2 or more) of active components.

The pharmaceutical ingredient may be in the form of a solution or as a solid - depending on the use and/or the mode of application and/or the mode of administration.

The lactobacilli may be used according to the present invention in any suitable form - whether when alone or when present in a combination with other components or ingredients. Likewise, combinations comprising the bacteria of the present invention and other componentsand/or ingredients (i.e. ingredients - such as food ingredients, functional food ingredients or pharmaceutical ingredients) may be used in any suitable form.

The lactobacilli may be used according to the present invention in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions.

Suitable examples of forms include one or more of: tablets, pills, capsules, ovules, solutions or suspensions, which may contain flavouring or colouring agents, for immediate-, delayed-, modified-, sustained-, pulsed- or controlled-release applications.

By way of example, if the bacteria of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch),sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included. Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethrai fatty acid esters, hydroxymethylcellulose, polyvinylpyrrolidone, and the like.

Preferred excipients for the forms include lactose, starch, a cellulose, milk sugar or high molecular weight polyethylene glycols.

For aqueous suspensions and/or elixirs, the bacteria of the present invention may be combined with various sweetening or flavouring agents, colouring matter or dyes, with emulsifying and/or suspending agents and with diluents such as water, propylene glycol and glycerin, and combinations thereof.

The forms may also include gelatin capsules; fibre capsules, fibre tablets etc.; or even fibre beverages.

Further examples of form include creams. For some aspects the microorganism used in the present invention may be used in pharmaceutical and/or cosmetic creams such as sun creams and/or after-sun creams for example.

In one aspect, the bacteria according to the present invention may be administered in an aerosol, for example by way of a nasal spray, for instance for administration to the respiratory tract.

### Prebiotics

In one embodiment, the use of the bacterium of the present invention may further comprise the use of one or more fibers/fibres and/or prebiotics.

Prebiotics are defined as a substrate that is selectively utilized by host microorganisms conferring a health benefit. These are generally ingredients that beneficially affect the health of the host by selectively stimulating the growth and/or activity of one or a limited number of bacteria (particularly, although not limited to lactobacilli and/or lactic acid bacteria), and thus improve host health. The prebiotic can be applied to oral route, but it can be also applied to other microbiologically colonized sites, for instance on the skin topically. Typically, prebiotics are carbohydrates (such as oligosaccharides), but the definition does not preclude non- carbohydrates, such as polyphenols, or polyunsaturated fatty acids or other ingredients that can be utilized selectively by a limited number of bacteria to confer a health benefit. The most prevalent forms of prebiotics are nutritionally classed as soluble fibers/fibres. To some extent, manyforms of dietary fibres exhibit some level of prebiotic effect.

In one embodiment, a prebiotic is a selectively fermented ingredient that allows specific changes, both in the composition and/or activity in the gastrointestinal or skin microflora that confers benefits upon host well-being and health.

Suitably, the prebiotic may be used according to the present invention in an amount of 0.01 to 100 g/day, preferably 0.1 to 50 g/day, more preferably 0.5 to 20 g/day. In one embodiment, the prebiotic may be used according to the present invention in an amount of 1 to 10 g/day, preferably 2 to 9 g/day, more preferably 3 to 8 g/day. In another embodiment, the prebiotic may be used according to the present invention in an amount of 5 to 50 g/day, preferably 10 to 25 g/day.

Examples of dietary sources of prebiotics include soybeans, inulin sources (such as Jerusalem artichoke, jicama, and chicory root), raw oats, unrefined wheat, unrefined barley and yacon.

Examples of suitable prebiotics include alginate, xanthan, pectin, locust bean gum (LBG), inulin, guar gum, galacto-oligosaccharide (GOS), fructo-oligosaccharide (FOS), polydextrose (i.e. Litesse^{®}), lactitol, L-Arabinose, D-Xylose, L-Rhamnose, D-Mannose, L-Fucose, inositol, sorbitol, mannitol, xylitol, fructose, carrageenan, alginate, microcrystalline cellulose (MCC), betaine, lactosucrose, soybean oligosaccharides, isomaltulose (Palatinose TM), isomalto-oligosaccharides, gluco-oligosaccharides, xylooligosaccharides, manno-oligosaccharides, beta-glucans, cellobiose, raffinose, gentiobiose, melibiose, xylobiose, cyciodextrins, isomaltose, trehalose, stachyose, panose, pullulan, verbascose, galactomannans, (human) milk oligosaccharides and all forms of resistant starches.

The combination of *Lactobacillus* and one or more fibres and/or prebiotics according to the present disclosure exhibits a synergistic effect in certain applications (i.e. an effect which is greater than the additive effect of the bacteria when used separately).

In one embodiment, the bacterium of the species *Ligilactobacillus salivarius, Lacticaseibacillus paracasei, Lacticaseibacillus rhamnosus,* or *Lactiplantibacillus plantarum* or a mixture thereof is used in combination with one or more fibres and/or prebiotic.

In one embodiment, the *Lactobacillus* or a mixture thereof used in combination with one or more fibres and/or prebiotic is the *Ligilactobacillus salivarius* Ls-33 (Ls-33).

In one the *Lactobacillus* or a mixture thereof used in combination with one or more fibres and/or prebiotic is the *Lacticaseibacillus paracasei* Lpc-37 (Lpc-37).

In one the *Lactobacillus* or a mixture thereof used in combination with one or more fibres and/or prebiotic is the *Lacticaseibacillus rhamnosus* HN001 (HN001).

In one the *Lactobacillus* or a mixture thereof used in combination with one or more fibres and/or prebiotic is the *Lactiplantibacillus plantarum* Lp-12407 (Lp-12407).

In one the *Lactobacillus* or a mixture thereof used in combination with one or more fibres and/or prebiotic is the *Lactiplantibacillus plantarum* Lp-12418 (Lp-12418).

Suitably, the prebiotic used is polydextrose, lactitol, inositol, L-Arabinose, D-Xylose, L-Rhamnose, D-Mannose, L-Fucose, sorbitol, mannitol, xylitol, fructose, carrageenan, alginate, microcrystalline cellulose (MCC), milk oligosaccharide or betaine.

In a further aspect, the invention relates to a composition, a skin care composition, dietary supplements, nutritional supplements, food products or a pharmaceutical acceptable composition comprising a bacterium of the species *Ligilactobacillus salivarius, Lacticaseibacillus paracasei, Lacticaseibacillus rhamnosus,* or *Lactiplantibacillus plantarum* or a mixture thereof and one or more fibres and/or a prebiotic.

In one aspect, the prebiotic can be a metabolite produced by a bacterium of species *Ligilactobacillus salivarius, Lacticaseibacillus paracasei, Lacticaseibacillus rhamnosus,* or *Lactiplantibacillus plantarum* or mixture thereof, and this metabolite can be utilized as cross- feeding of other microbes, for instance when applied topically on the skin, affecting the health beneficially.

In one aspect, the prebiotic can be a metabolite produced by a bacterium of strain *Ligilactobacillus salivarius* Ls-33 (Ls-33).

In one aspect, the prebiotic can be a metabolite produced by a bacterium of strain *Lacticaseibacillus paracasei* Lpc-37 (Lpc-37).

In one aspect, the prebiotic can be a metabolite produced by a bacterium of strain *Lacticaseibacillus rhamnosus* HN001 (HN001).

In one aspect, the prebiotic can be a metabolite produced by a bacterium of strain *Lactiplantibacillus plantarum* Lp-12407 (Lp-12407).

In one aspect, the prebiotic can be a metabolite produced by a bacterium of strain *Lactiplantibacillus plantarum* Lp-12418 (Lp-12418).

### Embodiments of the invention

For the avoidance of doubt, some of the embodiments the present invention relates to are set out below:
Embodiment 1: Non-therapeutic use as defined in the appended claims.
Embodiment 2. The non-therapeutic use as defined in the appended claims.

### Examples

### Materials and methods

Five DuPont Nutrition Biosciences ApS probiotic strains, *Ligilactobacillus salivarius* Ls-33, *Lacticaseibacillus paracasei* Lpc-37, *Lacticaseibacillus rhamnosus* HN001, *Lactiplantibacillus plantarum* Lp-12407 and *Lactiplantibacillus plantarum* Lp-12418, were tested in *in vitro* with Normal Human Epidermal Keratinocyte (NHEK) cells for their effects on skin hydration, moisturization and anti-aging properties. The probiotic strains were tested as live, lysed or as a postbiotic solution with NHEK cells with and without hyperosmotic stress (NaCl).

The effects of the strains on NHEK cells were investigated first by cell viability and confluency measurements. In addition to this, the effects of the strains on NHEK cells were studied measuring the amount of produced selected molecules: IL-1a, IL-6 and IL-8.

### NHEK cells

Adult normal human epidermal keratinocytes (NHEKs) (Gibco^{™}) were maintained in EpiLife^{®} medium with 60 µM calcium (Gibco^{™}), supplemented with Human Keratinocyte Growth Supplement (HKGS) (Gibco^{™}) at 5 % CO₂ atmosphere, +37°C. For the experiments, the earliest possible passage was utilized.

### Probiotic cell culture and test sample preparations

*Ligilactobacillus salivarius* strain Ls-33 (DSM 33831, DGCC9868) (commercially available from DuPont Nutrition Biosciences ApS), *Lacticaseibacillus paracasei* strain Lpc-37 (DSM 32661, DGCC4981) (commercially available from DuPont Nutrition Biosciences ApS), *Lacticaseibacillus rhamnosus* strain HN001 (DSM 22876, DGCC1460) (commercially available from DuPont Nutrition Biosciences ApS), *Lactiplantibacillus plantarum* strain Lp-12407 (DSM 32654, DGCC12407) (commercially available from DuPont Nutrition Biosciences ApS) and *Lactiplantibacillus plantarum* strain Lp-12418 (DSM 32655, DGCC12418) (commercially available from DuPont Nutrition Biosciences ApS) were cultured anaerobically at +37°C in MRS broth (Neo-gen). For studies with live and lysed bacteria, and postbiotics, the bacteria were grown until exponential growth phase was reached. Bacterial cells and soluble metabolites were separated by centrifuging at +4°C, 5 min, 4000 rpm. Bacterial pellets (cells) were resuspended to either supplemented EpiLife^{®} cell culture medium or differentiation medium (DM). The bacterial cell densities were determined with optical density meter (BioPhotometer, Eppendorf) and flow cytometry (FACSCalibur, Becton Dickinson, San Jose, CA, USA). Bacterial cell lysates were prepared by shearing the cells with Precellys 24 bead beater (Bertin Technologies, Saint-Quentin-en-Yvelines Cedex, France) with grinding kit VK01 (Bertin Technologies) at 6800 rpm with 3 cycles of 30 seconds. The breakdown of the cells was confirmed by light microscopy. The bacterial cell lysates were sterile filtered with 0.2 µm syringe units (Sartorius) before applying them to keratinocytes. The live and lysed bacterial cells were administered as 100 bacterial cells towards one keratinocyte cell. The soluble metabolites were diluted to 10 % (vol/vol) in either supplemented EpilLife^{®} cell culture medium or to differentiation medium (DM), and sterile filtered with 0.2 µm syringe units (Sartorius) before applying them to cells.

### Stress conditions

Hyperosmotic stress for keratinocyte studies was induced by adding NaCl (J.T. Baker) to supplemented EpiLife^{®} cell culture medium or to differentiation medium (DM), to reach 150 mM molar concentration, which corresponds about 570 mOsm/kg osmolality measured by osmometer (Ordior). The stresses were applied to the cells with the bacterial test solutions, except with differentiated keratinocytes, for which the hyperosmotic stress was applied topically whereas the bacterial test solutions were on the basolateral side.

### MTT and confluency assays

MTT 3-(4,5-dimethylthiazol-2-yl)-2,5-diphenyltetrazolium bromide assay was used to evaluate the metabolic activity (viability) of the cells after the test sample incubation. Briefly, to assess the metabolic activity of NHEK cells, 10⁴ cells were plated in a well of 96-well plate (Nunc^{™} MicroWell^{™}, Thermo Scientific) and after 24 h test samples in 0,1 ml volume were applied topically. After 24 h incubation with NHEK cells, the test samples were removed, cells were washed twice with phosphate buffered saline (PBS) and treated with 0,5 mg/ml MTT (Sigma-Aldrich) -reagent solution (diluted in DMEM, Gibco) for 2 hours at +37°C, 5 % CO₂ atmosphere. MTT-solution was collected, and replaced with 0,12ml of dimethyl sulfoxide (DMSO) (Sigma-Aldrich) and incubated in orbital shaker for 5 min protected from light. Volume of 0,09 ml of the supernatant was transferred to a 96-well plate and the absorbances of the samples were analysed with EnSight plate reader (Perkin Elmer, USA) measuring the 570 nm absorbance (reducing the background absorbance with 700 nm).

Confluency measurement was used to assess the cell morphology-related information by determining the area covered by cells. Briefly, to assess the cell confluency of NHEK cells, 10⁴ cells were plated in a well of 96-well plate (Nunc^{™} MicroWell^{™}, Thermo Scientific) and after 24 h to let the cells to attach at +37°C, 5 % CO₂ atmosphere, test samples in 0,1 ml volume were applied topically. After 24 h incubation with NHEK cells at +37°C, 5 % CO₂ atmosphere, the test samples were removed, and cells were washed twice with phosphate buffered saline (PBS). Brightfield imaging with EnSight plate reader (Perkin Elmer, USA) and the accompanied software (Kaleido, Perkin Elmer, USA) was used to determine the cell confluency.

### ELISA assays

Multiplex sandwich ELISA assays (Quanterix) were used to analyze the amount of produced human cytokines interleukin IL-1a, IL-6 and IL-8 from selected samples collected.

A transwell system creating two compartments was used with differentiated keratinocytes to determine the produced biomarkers with and without topical stress condition. To differentiate the keratinocytes, the cells were plated on ThinCert^{™} cell culture inserts (Greiner Bio-one) at density of 10⁵ cells/cm². After 48 h incubation at +37°C, in 5 % CO₂ atmosphere the medium was aspirated and changed to differentiation medium (DM) consisting of EpiLife^{®} medium with 1,45 mM CaCl₂ supplemented with HKGS and incubated for 3 days at 5 % CO₂ atmosphere.

At third day after starting the differentiation the media was changed both from basal and topical side to fresh DM and the differentiation checked by measuring the transepithelial electrical resistance (TEER) by cellZscope device (nanoAnalytics GmbH). The cells were thereafter incubated further over night at +37°C, in 5 % CO₂ atmosphere before initiating the experiment. Test samples in 1,5 ml volume were applied basolaterally and hyperosmotic stress topically in 0,5 ml volume. After 24 h incubation at +37°C, in 5 % CO₂ atmosphere, samples for ELISA assays were collected from the basolateral side and stored frozen at -80°C until analysis.

All ELISA biomarkers were analysed from the collected cell culture samples according to the manufacturer's instructions.

### Statistical analyses

Regarding MTT and confluency assays as well as ELISA assays for biomarkers IL-1a, IL-6 and IL-8, the statistical significances between the treatments were determined with GraphPad Prism version 9.2.0. using one-way ANOVA and Dunnett's multiple comparisons test, considering p-values of 0.05 or less as significant.

The result bar plots show mean ± standard deviation.

### RESULTS

### Metabolic activity

The metabolic activity of the cells describes the general well-being of the cells. The MTT method measures the cellular metabolic activity of living cells, and the reduction potential of a cell or the availability of reducing compounds to drive cellular energetics. It is also utilized to measure viability of the cells. Absorbance values greater than the control indicate cell proliferation or higher metabolic activity, while lower values suggest cell death or inhibition of proliferation or lower metabolic activity. The decrease in MTT activity in cells is considered an indicator of cell redox activity. Senescent cells, or cells that are not able to divide normally accumulate in chronologically aged and photoaged skin; and may contribute to age-related skin changes and pathologies. Epidermal thinning, that is associated with aging skin, is, in part, caused by decreased proliferation and renewal capacity of basal keratinocytes and reduced epidermal stem cell number. Also, the dermal-epidermal junction (DEJ) that connects the epidermis to the underlying dermis as well as the dermis itself become thinner. Senescent cells lose their functionality and affect also surrounding cells by secreting various molecules, including pro-inflammatory chemokines, cytokines and various proteases.

Hydration status of the skin cells is crucial for their metabolic activity. Thus, one of the major functions of the skin is to provide a protection against dehydration and water loss. The skin of terrestrial animals, including humans, is exposed to an environment that varies in humidity from 0 to 100% relative humidity. Less than 100% relative humidity can produce significant dehydration or osmotic stress on the epidermis, which then must respond and protect the organism from water loss. Aged skin is drier than younger skin and has more rigid epidermis. Wrinkles are formed through the folding of epidermis along with bulk deformation of dermis, dehydrated rigid epidermis resists more to the folding stress, and hence shows less wavy surface, which means that dehydration generates wrinkles with larger amplitudes and periods. With an adequate amount of water in the *stratum corneum* (SC), the skin maintains its intact barrier function, feels soft and flexible and looks smooth and healthy. Drier skin shows more wrinkles and deeper furrows with wider intervals.

### NHEK cell metabolic activity - NaCl stress

The metabolic activity of NHEK cells under stress was screened with MTT (3-(4,5-dimethylthiazol-2-yl)-2,5-diphenyltetrazolium bromide) assay after incubating the cells with test solutions with hyperosmotic stress (induced by NaCl) for 24 hours. The number of viable cells present is reflected in the capability of NAD(P)H-dependent cellular oxidoreductase enzymes to reduce the MTT reagent to insoluble formazan, which can be measured by absorbance analysis. Hyperosmotic stress disturbs the cell osmotic balance and was used to model dehydration.

During hyperosmotic stress induced by NaCl, Ls-33 as live (32.30%, p=0.0027) and postbiotic form (32.86%, p=0.0011), live Lp-12407 bacteria (34.27%, p=0.0001), live Lpc-37 bacteria (47.75%, p<0.0001), postbiotic form of Lp-12418 (32.41%, p=0.0023), and HN001 as live (35.18%, p<0.0001) and postbiotic form (30.33%, p=0.0401) were able to increase the metabolic activity (viability) of NHEK cells compared to NaCl treated control cells (23.88%) (Figure 1).

Hyperosmotic stress causes metabolic decrease in the cells and can cause cell senescence. Probiotic strains Ls-33, Lp-12407, Lpc-37, Lp-12418 and HN001 were able to slow down the reduction of metabolic activity and/or cell viability caused by hyperosmotic stress, which might refer to the ability to support the cell proliferation and viability and equalize the osmotic difference in the skin during dehydration that can induce the signs of aging.

### Confluency

The visual inspection of the cells and measurement of confluency determines the percentual image area covered by cells, or the area from the culture dish that is covered by the cells. Increase in the confluency indicates either that the cell number has increased, or that cells are spreading more or adhere better to the substratum, or that the volume of the cells has increased. Epithelial sheet spreading is a fundamental cellular process that must be coordinated with cell division and differentiation to restore tissue integrity. Dividing keratinocytes are committed to differentiation, when the cell confluency with increased cellular density is high enough. Senescent cells present in aged skin cannot divide and for that reason it is of benefit that the confluency increases.

In hyperosmotic stress, cell volume is reduced, cell shrinkage occurs as a result of water efflux. In aged skin the blood circulation in dermis and expression of osmolyte transporters is reduced leading to reduced recovery of the cell volume from the osmotic stress. Hyperosmotic stress has been also implicated in the pro-inflammatory signaling and thus in promotion of aging. It causes cessation of all major biosynthetic routes, including DNA synthesis and repair, transcription, protein translation and degradation, as well as mitochondrial function. As a result, cell cycle progression and cell proliferation are halted. There is a concomitant increase in oxidative stress and activation of apoptotic pathways, which might lead to reduction in cell number.

### NHEK cell confluency- No stress

After incubating the cells with test solutions for 24 hours, confluency, the area covered by the cells, was assessed with brightfield imaging and the accompanied analysis software.

Live Lp-12407 bacteria (44.03%, p=0.0461) as well as postbiotic form of Lpc-37 bacteria (44.65%, p=0.0171) had statistically significant increases in the NHEK cell confluency compared to medium control sample (38.45%) (Figure 2).

The slight increase in the confluency might reflect the increase in cell amounts and adherence to the well substratum. In favorable conditions cells maintain, and even increase, the proliferation with a simultaneous balance in the cell volume regulation.

### NHEK cell confluency - NaCl stress

After 24h incubation in the presence of hyperosmotic stress, which disturbs the cells' osmotic balance, the area covered by the cells, confluency, was assessed with brightfield imaging and the accompanied analysis software. The confluency analysis reveals interesting information especially under hyperosmotic conditions, as it causes cell shrinkage when they try to balance the osmotic difference.

During the NaCl stress, several test compounds were able to restore the cell confluency compared to NaCl control sample (15.84%), among the others Lp-12407 as live (32.77%, p<0.0001) and in postbiotic form (22.83%, p=0.0351), Lpc-37 as live (31.00%, p<0.0001), Lp-12418 as live (30.89%, p<0.0001) and lysed (22.95%, p=0.0304), and HN001 as live (24.66%, p=0.0032) (Figure 3). Also, Ls-33 as live (21.92%) had some increasing effect in the confluency compared to the hyperosmotic stress control (Figure 3).

All the strains, Ls-33, Lp-12407, Lpc-37, Lp-12418 and HN001, were able to counteract the harmful effects of the hyperosmotic condition, as they were able to attenuate the cell shrinkage and cell detaching that was observed as reduction in the cell confluency with NaCl stress control samples. This might refer to the ability of the strains, Lp-12407, Lpc-37, Lp-12418 and HN001 to support skin cells during osmotic stress and in dehydration that can cause skin aging.

### NHEKS biomarkers - no stress and hyperosmotic stress

### Interleukin-1 alpha (IL-1a)

During the exposure of keratinocytes stressed with hyperosmotic stress, all strains and forms, live (p<0.0001), lysed (p=0.0166) and postbiotic (p<0.0001) form of Ls-33; live (p <0.0001), lysed (p=0.0061) and postbiotic (p<0.0001) form of Lp-12407; live (p <0.0001), lysed (p=0.0021) and postbiotic (p<0.0001) form of Lpc-37; live (p<0.0001), lysed (p=0.0006) and postbiotic (p<0.0001) form of Lp-12418; as well as live (p<0.0001), lysed (p=0.0024) and postbiotic (p<0.0001) form of HN001, increased the expression of IL-1α significantly compared to control (Figure 4).

Keratinocytes produce IL-1α constitutively, and expression of these has protective function through stimulation of keratinocyte proliferation and differentiation. IL-1α also stimulates expression of other cytokines, such as IL-6, and IL-8. Especially IL-1α levels has been shown to be decreased in aged skin. IL-1α has also role in the stimulating and enhancing in the lipid synthesis in the keratinocytes, that contribute the formation of the lipids that are part of the skin barrier. When skin barrier was perturbed with tacrolimus in a mouse model, IL-1α increased the barrier recovery and restored permeability and antimicrobial barrier. In addition, it has been shown to upregulate the expression of genes associated to cell adhesion, proliferation and junction formation, such as occludin and claudin-1. IL-1α stimulation in aged murine epidermis improves barrier homeostasis. Therefore, the increase in IL-1α by the probiotics increases viability and barrier repair and maintenance in the epidermis. Thus, the increase in IL-1α would improve the skin barrier function in many ways benefiting the aged skin.

### Interleukin-6 (IL-6)

During the exposure of keratinocytes without stress, live (p<0.0001) and postbiotic (p<0.0001) form of Ls-33; live (p=0.0005) and postbiotic (p<0.0001) form of Lp-12407; and live (p<0.0001) and postbiotic (p<0.0001) form of Lpc-37, were all able to increase IL-6 production significantly compared to control.

During the exposure of keratinocytes stressed with hyperosmotic stress, live (p<0.0001) and postbiotic (p=0.0006) form of Ls-33; all forms, live (p<0.0001), lysed (p<0.0001) and postbiotic (p<0.0001) of Lp-12407; live (p<0.0001) and postbiotic (p<0.0001) form of Lpc-37; as well as live Lp-12418 (p=0.0003) and live HN001 (p=0.0015) strains increased the expression of IL-6 significantly compared to control (Figure 5; Figure 6).

IL-6 can be induced by multiple stimuli, including UVB radiation, TLR stimulation and various proinflammatory cytokines. IL-6 promotes keratinocyte proliferation, skin thickening and skin barrier repair. Topical IL-6 application has been shown to improve the skin barrier repair after mechanical injury. Especially, in aging skin epithelial proliferation is desired to maintain the basal and the differentiating populations in balance. The increase in IL-6 indicates improvement in the capacity of NHEKs to proliferate and repair the skin barrier benefiting the aged skin. Interleukin-8 (IL-8) (also known as chemokine CXCL8)

During the exposure of keratinocytes without stress, live forms of strains Ls-33 (p<0.0001), Lp-12407 (p=0.0088), Lpc-37 (p<0.0001), and HN001 (p=0.0232) were all able to increase IL-8 production significantly compared to control.

During the exposure of keratinocytes stressed with hyperosmotic stress, live (p= 0.0003), lysed (p <0.0001) and postbiotic (p<0.0001) form of Ls-33; live (p<0.0001), lysed (p<0.0001), and postbiotic (p<0.0001) form of Lp-12407; live (p<0.0001), lysed (p<0.0001), and postbiotic (p<0.0001) form of Lpc-37; live (p<0.0001), lysed (p=0.0239) and postbiotic (p<0.0001) form of Lp-12418, and live (p=0.0002) and postbiotic (p<0.0001) form of HN001, increased the expression of IL-8 significantly compared to control (Figure 7).

Chemokines, including IL-8 regulate proliferation and differentiation of normal keratinocytes. IL-8 is expressed during injury and can be induced by proinflammatory cytokines, such as IL-1 or TNF-α. It stimulates keratinocyte migration and proliferation. Especially, in aging skin epithelial proliferation is desired to maintain the basal and the differentiating populations in balance. Thus, increase in IL-8 indicates improvement in the capacity of NHEKs to proliferate and repair skin barrier benefiting the aged skin.

## Claims

1. Non-therapeutic use of a bacterium selected from strain *Ligilactobacillus salivarius* Ls-33 (Ls-33), strain *Lacticaseibacillus paracasei* Lpc-37 (Lpc-37), strain *Lacticaseibacillus rhamnosus* HN001 (HN001), strain *Lactiplantibacillus plantarum* Lp-12407 (Lp-12407) and strain *Lactiplantibacillus plantarum* Lp-12418 (Lp-12418), or a mixture thereof for preventing, reducing or treating skin aging in a subject.

2. The non-therapeutic use according to claim 1, wherein the bacterium or the mixture thereof maintains metabolic activity/cell viability in the skin, increases the metabolic activity/cell viability in the skin or slows down the reduction of metabolic activity and/or cell viability in the skin.

3. The non-therapeutic use according to claim 1, wherein the bacterium or the mixture thereof maintains and stimulates epidermal skin natural and intrinsic homeostatic cytokine production of keratinocytes.

4. The non-therapeutic use according to claim 3, wherein the maintenance and stimulation of the epidermal skin natural and intrinsic homeostatic cytokine production of keratinocytes maintains a metabolic activity/cell viability in the skin, increases the metabolic activity/cell viability in the skin or slow down the reduction of metabolic activity and/or cell viability in the skin.

5. The non-therapeutic use according to any one of claims 1 to 4, wherein the bacterium or the mixture thereof is at least one component of a composition, a skin care composition, a dietary supplement, a nutritional supplement, a food product or a pharmaceutical acceptable composition.

6. Bacterium selected from strain *Ligilactobacillus salivarius* Ls-33 (Ls-33), strain *Lacticaseibacillus paracasei* Lpc-37 (Lpc-37), strain *Lacticaseibacillus rhamnosus* HN001 (HN001), strain *Lactiplantibacillus plantarum* Lp-12407 (Lp-12407) and strain *Lactiplantibacillus plantarum* Lp-12418 (Lp-12418) or a mixture thereof for use in preventing, reducing or treating skin aging in a subject.

7. The bacterium for use according to claim 6, wherein the bacterium or the mixture thereof maintains metabolic activity/cell viability in the skin, increases the metabolic activity/cell viability in the skin or slows down the reduction of metabolic activity and/or cell viability in the skin.

8. The bacterium for use according to claim 6, wherein the bacterium or the mixture thereof maintains and stimulates epidermal skin natural and intrinsic homeostatic cytokine production of keratinocytes.

9. The bacterium for use according to claim 8, wherein the maintenance and stimulation of the epidermal skin natural and intrinsic homeostatic cytokine production of keratinocytes maintains a metabolic activity/cell viability in the skin, increases the metabolic activity/cell viability in the skin or slow down the reduction of metabolic activity and/or cell viability in the skin.

## Patentansprüche

1. Nicht-therapeutische Verwendung eines Bakteriums, ausgewählt aus Stamm Ligilactobacillus salivarius Ls-33 (Ls-33), Stamm Lacticaseibacillus paracasei Lpc-37 (Lpc-37), Stamm Lacticaseibacillus rhamnosus HN001 (HN001), Stamm Lactiplantibacillus plantarum Lp-12407 (Lp-12407) und Stamm Lactiplantibacillus plantarum Lp-12418 (Lp-12418) oder einer Mischung davon zum Vorbeugen, Verringern oder Behandeln von Hautalterung bei einem Individuum.

2. Nicht-therapeutische Verwendung nach Anspruch 1, wobei das Bakterium oder die Mischung davon die Stoffwechselaktivität/Zellviabilität in der Haut aufrechterhält, die Stoffwechselaktivität/Zellviabilität in der Haut erhöht oder die Verringerung der Stoffwechselaktivität und/oder Zellviabilität in der Haut verlangsamt.

3. Nicht-therapeutische Verwendung nach Anspruch 1, wobei das Bakterium oder die Mischung davon die natürliche und intrinsische homöostatische Zytokinproduktion von Keratinozyten der epidermalen Haut aufrechterhält und stimuliert.

4. Nicht-therapeutische Verwendung nach Anspruch 3, wobei die Aufrechterhaltung und Stimulation der natürlichen und intrinsischen homöostatischen Zytokinproduktion von Keratinozyten der epidermalen Haut eine Stoffwechselaktivität/Zellviabilität in der Haut aufrechterhält, die Stoffwechselaktivität/Zellviabilität in der Haut erhöht oder die Verringerung der Stoffwechselaktivität und/oder Zellviabilität in der Haut verlangsamt.

5. Nicht-therapeutische Verwendung nach einem der Ansprüche 1 bis 4, wobei das Bakterium oder die Mischung davon mindestens eine Komponente einer Zusammensetzung, einer Hautpflegezusammensetzung, eines Nahrungsergänzungsmittels, eines Nahrungsergänzungsmittels, eines Lebensmittelprodukts oder einer pharmazeutisch unbedenklichen Zusammensetzung ist.

6. Bakterium, ausgewählt aus Stamm Ligilactobacillus salivarius Ls-33 (Ls-33), Stamm Lacticaseibacillus paracasei Lpc-37 (Lpc-37), Stamm Lacticaseibacillus rhamnosus HN001 (HN001), Stamm Lactiplantibacillus plantarum Lp-12407 (Lp-12407) und Stamm Lactiplantibacillus plantarum Lp-12418 (Lp-12418) oder einer Mischung davon zur Verwendung beim Vorbeugen, Verringern oder Behandeln von Hautalterung bei einem Individuum.

7. Bakterium zur Verwendung nach Anspruch 6, wobei das Bakterium oder die Mischung davon die Stoffwechselaktivität/Zellviabilität in der Haut aufrechterhält, die Stoffwechselaktivität/Zellviabilität in der Haut erhöht oder die Verringerung der Stoffwechselaktivität und/oder Zellviabilität in der Haut verlangsamt.

8. Bakterium zur Verwendung nach Anspruch 6, wobei das Bakterium oder die Mischung davon die natürliche und intrinsische homöostatische Zytokinproduktion von Keratinozyten der epidermalen Haut aufrechterhält und stimuliert.

9. Bakterium zur Verwendung nach Anspruch 8, wobei die Erhaltung und Stimulation der natürlichen und intrinsischen homöostatischen Zytokinproduktion von Keratinozyten der epidermalen Haut eine Stoffwechselaktivität/Zellviabilität in der Haut aufrechterhält, die Stoffwechselaktivität/Zellviabilität in der Haut erhöht oder die Verringerung der Stoffwechselaktivität und/oder Zellviabilität in der Haut verlangsamt.

## Revendications

1. Utilisation non thérapeutique d'une bactérie choisie parmi la souche Ligilactobacillus salivarius Ls-33 (Ls-33), la souche Lacticaseibacillus paracasei Lpc-37 (Lpc-37), la souche Lacticaseibacillus rhamnosus HN001 (HN001), la souche Lactiplantibacillus plantarum Lp-12407 (Lp-12407) et la souche Lactiplantibacillus plantarum Lp-12418 (Lp-12418), ou un mélange de celles-ci pour prévenir, réduire ou traiter le vieillissement de la peau chez un sujet.

2. Utilisation non thérapeutique selon la revendication 1, dans laquelle la bactérie ou son mélange maintient l'activité métabolique/viabilité cellulaire dans la peau, augmente l'activité métabolique/viabilité cellulaire dans la peau ou ralentit la réduction de l'activité métabolique et/ou de la viabilité cellulaire dans la peau.

3. Utilisation non thérapeutique selon la revendication 1, dans laquelle la bactérie ou son mélange maintient et stimule la production de cytokines homéostatiques naturelles et intrinsèques de la peau épidermique de kératinocytes.

4. Utilisation non thérapeutique selon la revendication 3, dans laquelle le maintien et la stimulation de la production de cytokines homéostatiques naturelles et intrinsèques de la peau épidermique de kératinocytes maintiennent une activité métabolique/viabilité cellulaire dans la peau, augmentent l'activité métabolique/viabilité cellulaire dans la peau ou ralentissent la réduction de l'activité métabolique et/ou de la viabilité cellulaire dans la peau.

5. Utilisation non thérapeutique selon l'une quelconque des revendications 1 à 4, dans laquelle la bactérie ou le mélange de celles-ci est au moins un composant d'une composition, d'une composition de soins de la peau, d'un supplément alimentaire, d'un supplément nutritionnel, d'un produit alimentaire ou d'une composition pharmaceutiquement acceptable.

6. Bactérie sélectionnée parmi la souche Ligilactobacillus salivarius Ls-33 (Ls-33), la souche Lacticaseibacillus paracasei Lpc-37 (Lpc-37), la souche Lacticaseibacillus rhamnosus HN001 (HN001), la souche Lactiplantibacillus plantarum Lp-12407 (Lp-12407) et la souche Lactiplantibacillus plantarum Lp-12418 (Lp-12418) ou un mélange de celles-ci pour une utilisation pour prévenir, réduire ou traiter le vieillissement de la peau chez un sujet.

7. Bactérie à utiliser selon la revendication 6, dans laquelle la bactérie ou son mélange maintient une activité métabolique/viabilité cellulaire dans la peau, augmente l'activité métabolique/viabilité cellulaire dans la peau ou ralentit la réduction de l'activité métabolique et/ou de la viabilité cellulaire dans la peau.

8. Bactérie à utiliser selon la revendication 6, dans laquelle la bactérie ou son mélange maintient et stimule la production de cytokines homéostatiques naturelles et intrinsèques de la peau épidermique de kératinocytes.

9. Bactérie à utiliser selon la revendication 8, dans laquelle le maintien et la stimulation de la production de cytokines homéostatiques naturelles et intrinsèques de la peau épidermique de kératinocytes maintiennent une activité métabolique/viabilité cellulaire dans la peau, augmentent l'activité métabolique/viabilité cellulaire dans la peau ou ralentissent la réduction de l'activité métabolique et/ou de la viabilité cellulaire dans la peau.
